# EUROPEAN PATENT SPECIFICATION

(11) **EP 2 186 507 B1**
(45) Date of publication and mention of the grant of the patent: **01.04.2015**
(21) Application number: 09002569.3
(22) Date of filing: 18.12.2002
(51) Int. Cl.: A61K 9/00, A61K 31/465, A61K 47/10

(54) **A liquid pharmaceutical formulation comprising nicotine for the administration to the oral cavity**
Flüssige pharmazeutische Formulierung mit Nikotin zur Abgabe in die Mundhöhle
Formule pharmaceutique liquide comportant de la nicotine pour l'administration de la cavité orale

(30) Priority: 27.12.2001 SE 0104388
(43) Date of publication of application: 19.05.2010
(62) Divisional of application: 02793665.7
(73) Proprietor: McNeil AB, 25109 Helsingborg (SE)
(72) Inventor: Lindell, Katarina, 241 93 Eslöv (SE); Bosson, Bengt, 256 55 Helsingborg (SE); Bergengren, Gunnar, 267 32 Bjuv (SE); Schlüter, Anette, 257 32 Rydebäck (SE)
(74) Representative: Potter Clarkson LLP

(56) References cited:
- EP-B1- 0 324 794
- WO-A1-99/55371
- GB-A- 2 255 892
- US-A- 4 579 858
- US-A- 5 656 255
- US-A- 5 721 257
- US-A- 5 955 098
- US-A- 6 024 097
- BECKETT A H ET AL: "Buccal absorption of basic drugs and its application as an in vivo model of passive drug transfer through lipid membranes", JOURNAL OF PHARMACY AND PHARMACOLOGY, ROYAL PHARMACEUTICAL SOCIETY OF GREAT BRITAIN, GB, vol. 19, 1 December 1967 (1967-12-01), pages 31S-41S, XP009142973, ISSN: 0022-3573
- BECKETT A H ET AL: "A POSSIBLE RELATION BETWEEN PKA1 AND LIPID SOLUBILITY AND THE AMOUNTS EXCRETED IN URINE OF SOME TOBACCO ALKALOIDS GIVEN TO MAN", JOURNAL OF PHARMACY AND PHARMACOLOGY, ROYAL PHARMACEUTICAL SOCIETY OF GREAT BRITAIN, GB, vol. 24, no. 2, 1 January 1972 (1972-01-01), pages 115-120, XP009060133, ISSN: 0022-3573
- AXELSSON A ET AL: "The anti-smoking effect of shewing gum with nicotine of high and low bioavailability", PROCEEDINGS OF THE WORLD CONFERENCE ON SMOKING AND HEALTH, XX, XX, 1 January 1975 (1975-01-01), pages 549-559, XP003000134,

## Description

### Technical Field

This invention relates to a liquid pharmaceutical formulation for delivering nicotine to a subject.

### Background of the Invention

### Tobacco dependence and reduction thereof

In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief active ingredient in the tobacco used in cigarettes, cigars, snuff and the like. Nicotine is also an addictive drug, though, and smokers characteristically display a strong tendency to relapse after having successfully stopped smoking for a time. Nicotine is the world's second most used drug, after caffeine from coffee and tea.

The main problem with tobacco smoking is its enormous implications on health. Today it is estimated that smoking-related diseases cause some 3 - 4 million deaths per year. In the US Surgeon General's 1988 report on The Health Consequences of Smoking, it was estimated that in the US alone about 300.000 deaths are caused each year by diseases related to cigarette smoking. In fact, excessive smoking is now recognized as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug.

The most advantageous thing a heavy smoker can do is to reduce or preferably even stop smoking completely. Experience shows, however, that most smokers find this extremely difficult since, mostly, tobacco smoking result in a dependence disorder or craving. The WHO has in its International Classification of Disorders a diagnosis called Tobacco Dependence. Others, like the American Psychiatric Association call the addiction Nicotine Dependence. It is generally accepted that these difficulties to stop smoking result from the fact that those heavy smokers are dependent on nicotine. The most important risk factors are, however, substances that are formed during the combustion of tobacco, such as carcinogenic tar products, carbon monoxide, aldehydes, and hydrocyanic acid.

### Effects of nicotine

The administration of nicotine can give satisfaction and the usual method is by smoking, either by smoking e g a cigarette, a cigar or a pipe, or by snuffing or chewing tobacco. However, smoking has health hazards and it is therefore desirable to formulate an alternative manner of administering nicotine in a pleasurable manner that can be used to facilitate withdrawal from smoking and/or used as a replacement for smoking.

Upon smoking of a cigarette, nicotine is quickly absorbed into the smoker's blood and reaches the brain within around ten seconds after inhalation. The quick uptake of nicotine gives the consumer a rapid satisfaction, or kick. The satisfaction, then, lasts during the time of smoking the cigarette and for a period of time thereafter. The poisonous, toxic, carcinogenic, and addictive nature of smoking has provided efforts for methods, compositions and devices, which help in breaking the habit of smoking.

Nicotine is an addictive poisonous alkaloid C₅H₄NC₄H₇NCH₃, derived from the tobacco plant. Nicotine is also used as an insecticide. Approximately forty milligrams of nicotine may kill an adult (Merck Index).

### Nicotine replacement products

One way to reduce smoking is to provide nicotine in a form or manner other than by smoking and some products have been developed to fulfill this need. Nicotine containing formulations are currently the dominating treatments for tobacco dependence.

The success in achieving reduction in the incidence of smoking has been relatively poor using presently known products. State of the art involves both behavioral approaches and pharmacological approaches. More than 80% of the tobacco smokers who initially quit smoking after using some behavioral or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market, such as nicotine chewing gums according to US 3,845,217. Several methods and means have been described for diminishing the desire of a subject to use tobacco, which comprises the step of administering to the subject nicotine or a derivative thereof as described in e g US 5,939,100 (nicotine containing microspheres) and US 4,967,773 (nicotine containing lozenge).

The effects of pH on the absorption of nicotine is discussed e g in Eur J Clin Pharmacol, Vol. 56,2001, pages 813 - 818, L. Molander et al, "Pharmacokinetic investigation of a nicotine sublingual tablet". The effects of pH on a liquid nicotine formulation for administration to the oral cavity are though not disclosed.

The use of skin patches for transdermal administration of nicotine has been reported (Rose, in Pharmacological Treatment of Tobacco Dependence, (1986) pp. 158 - 166, Harvard Univ. Press). Nicotine-containing skin patches that are in wide use today can cause local irritation and the absorption of nicotine is slow and affected by cutaneous blood flow.

Nicotine-containing nose drops have been reported (Russell et al., British Medical Journal, Vol. 286, p. 683 (1983); Jarvis et al., British Journal of Addiction, Vol. 82, p. 983 (1987)). Nose drops, however, are difficult to administer and are not convenient for use at work or in other public situations. Ways of administrating nicotine by way of delivery directly into the nasal cavity by spraying is known from US 4,579,858, DE 32 41437 and WO/93 127 64. There may, though, be local nasal irritation with use of nasal nicotine formulations. The difficulty in administration also results in unpredictability of the dose of nicotine administered.

Also, inhaling devices resembling a cigarette are known for uptake of nicotine vapors as suggested in US 5,167,242. An aerosol for deposing nicotine in the lungs is disclosed in DE 32 41 437.

Mouth sprays comprising nicotine are known in the art, e g according to US 6,024,097 wherein is disclosed a method of assisting a smoker in giving up the smoking habit whereby is used a plurality of aerosol dispensers comprising progressively lesser concentrations of nicotine. The aerosol is intended to be administered in to the mouth. The liquid in the dispensers essentially consists of nicotine and alcohol.

A similar mouth spray is disclosed in US 5,810,018, whereby in addition the aerosol comprises progressively greater concentrations of at least one selected stimulant.

WO 98/24420 discloses an aerosol device with an active and a propellant. The device may be used for e g sublingual administration. Nicotine is mentioned as an active in a long "laundry list" of drugs. There are though no examples on nicotine formulations.

US 5,721,257 discloses a method for treating a condition responsive to nicotine therapy comprising a first treatment with transdermally administered nicotine and a second treatment with transmucosally administered nicotine. It is stated that the transmucousal administration may be accomplished via an aerosol to the nasal membranes. No administration to the oral cavity is disclosed.

WO 97/38663 discloses a buccal aerosol spray using a non-polar solvent. Nicotine is mentioned as one useful active in this spray.

US 5,955,098 likewise discloses a buccal non-polar spray wherein nicotine may be an active.

None of the known mouth sprays comprise any buffering.

### Prior art and problems thereof

The captioned means and methods do not satisfy the craving that certain users of tobacco experience. Specifically these means and methods generally do not provide for a sufficiently rapid uptake of nicotine without adverse effects.

This means that none of the hitherto known means and methods satisfactorily fulfills the following well-known NRT teaching by Russel et al:
I: A fast delivery or "boost" of nicotine, sufficiently rapid to give positive subjective nicotine effects in contrast with current nicotine gums and patches, will lead to faster craving relief, and
II: faster craving relief will give better craving control, and
III: better craving control should result in higher overall quit rates.

For the captioned see Russel, M.A.H., Stapleton, J.A. and Feyerabend C. Nicotine boost per cigarette as the controlling factor of intake regulation of smokers; In: Clark et al. (Eds.) Effects of Nicotine on Biological Systems II, Advances in Pharmacological Sciences, Birkhauser Verlag, Basel, (1995) 233-238.

In light of the aforementioned problems there is a strong need and interest to develop means and methods for the administration of nicotine to provide a fast satisfaction to a person craving for nicotine or to provide a sense of smoking satisfaction without smoking, whereby also may be avoided problems associated with the prior art means and methods. The present invention addresses said need and interest.

### Summary of the Invention

In view of the foregoing disadvantages known in the art when trying to deliver nicotine to a subject so as to obtain a rapid transmucosal uptake of nicotine in the oral cavity of the subject the present invention provides a new and improved product, systems and methods for obtaining a rapid transmucosal uptake of nicotine in the oral cavity of the subject.

Objects of the present invention are to provide an efficient and effective product, as well as methods and systems for a rapid uptake of nicotine in a subject to avoid the disadvantages of previously known products and methods. The present invention also satisfactorily satisfies the above teaching of Russel et al.

The present invention provides a pharmaceutical formulation for use in the treatment of addiction to tobacco or nicotine as defined in the accompanying claims.

Thus, the disclosure invention provides a method for delivering nicotine to a subject comprising administering to a subject a liquid pharmaceutical formulation of the invention into the oral cavity of the subject and allowing the nicotine to be absorbed into the systemic circulation of the subject essentially by rapid buccal uptake of nicotine as well as a method for manufacturing said liquid pharmaceutical formulation.

The present disclosure also provides a method for obtaining reduction of the urge to smoke or use tobacco containing material and/or for providing a fast sense of smoking satisfaction without smoking, comprising the steps of replacing at least partly the tobacco containing material with said liquid pharmaceutical formulation of the invention,
administering to a subject the liquid pharmaceutical formulation into the oral cavity of the subject and allowing the nicotine to be systemically absorbed by the subject essentially by buccal uptake of nicotine.

Furthermore, the present disclosure provides a system for delivering nicotine to a subject, comprising said liquid pharmaceutical formulation and at least one other means for obtaining reduction of the urge to smoke or use of tobacco as well as a system for obtaining reduction of the urge to smoke or otherwise use of tobacco and/or for providing a sense of smoking satisfaction without smoking, comprising a liquid pharmaceutical formulation as per above and at least one other method for obtaining reduction of the urge to smoke or otherwise use tobacco. Said system may be a system wherein the at least one other method is selected from the group consisting of administration through chewing gums, nasal sprays, transdermal patches, inhaling devices, lozenges, tablets and parenteral methods, subcutaneous methods, intravenous methods, rectal methods, vaginal methods and transmucosal methods; or other use of tobacco.

The present invention provides for a flexible, convenient and discrete use in comparison with other means for transmucosal delivery of nicotine, e g chewing gums, lozenges and tablets. No chewing or sucking is necessary. Further and in contrast to other transmucosal dosage forms the present liquid pharmaceutical formulation provides nicotine in a form being directly buccally absorbable by a subject. Known formulations for nasal delivery of nicotine are inconvenient - side effects include running nose, nasal irritation and irritation of the eyes. The nicotine in chewing gums, lozenges and tablets need pass a transformation phase, involving e g mastication, disintegration, melting and/or dissolution, prior to being present in a directly absorbable form. A nicotine patch provides for a discrete administration, but does not provide for a fast uptake of nicotine.

A product according to the present invention is alkalized by buffering in such a way that upon administration of the liquid pharmaceutical formulation the pH of the liquid of the oral cavity is increased by 0.3 - 4 pH units, or preferably increased by 0.5 - 2.5 pH units.

Use of said product will according to the invention rapidly delivers nicotine to a subject and will also provide for obtaining a quick and/or sustained and/or complete reduction of the urge to smoke or use tobacco and/or for providing a sense of smoking satisfaction without smoking resembling the sense of smoking satisfaction obtained after regular smoking or use of tobacco.

### Legend of Figures

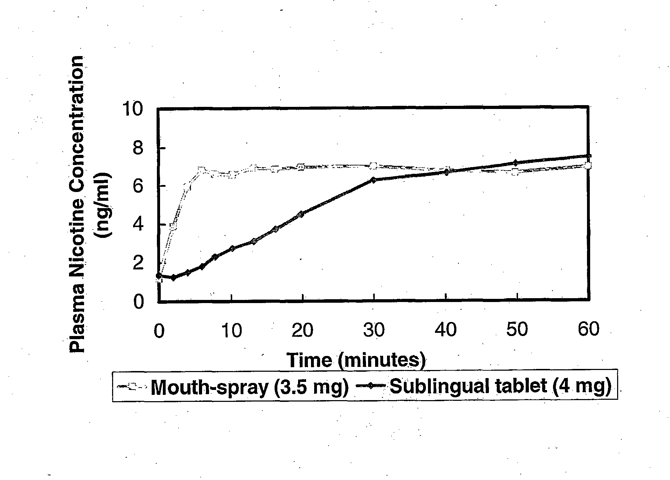

Figure 1 is a diagram showing venous blood plasma level concentrations of nicotine after two different ways of administering nicotine. For both ways of administration one unit dose was administered at time zero. No further doses were administered. 50 persons, all being nicotine users, took part in this test. "Spray" represents 200 µl of a liquid pharmaceutical formulation according to below Example 4 being sprayed under the tongue. This unit dose comprised 3.5 mg nicotine measured as free base. "Microtab" represents one tablet of Nicorette^{®} Microtab, comprising 4 mg nicotine measured as free base. Nicorette^{®} Microtab is pharmacologically equivalent to Nicorette^{®} Gum. "Spray" comprises a buffer. "Microtab" comprises no buffer. With "Spray" the liquid pharmaceutical formulation was held in the mouth for one minute before swallowing. With "Microtab" the tablet was kept under the tongue until dissolved. Each symbol on the respective graph represents one measurement of nicotine in venous blood plasma.

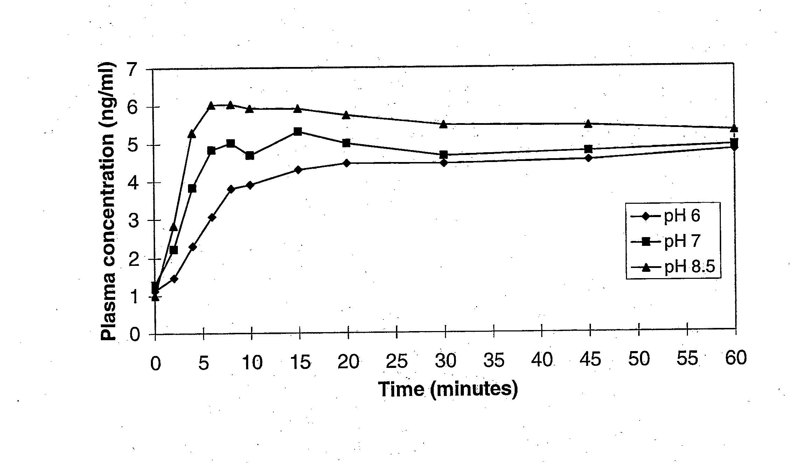

Figure 2 shows mean plasma concentrations after sublingual administration of three liquid pharmaceutical formulations with pH 6, 7 and 8.5 respectively. For each formulation 200 µl was sprayed under the tongue at time zero. For all said three formulations the concentration of nicotine was 10 mg/ml, i e each 200 µl spray dose as above contained 2 mg nicotine calculated as free base. The formulation with pH 8.5 was a formulation according to below Example 1. The formulations with pH 7 and pH 6 were formulations according to below Example 2 and Example 3 respectively.

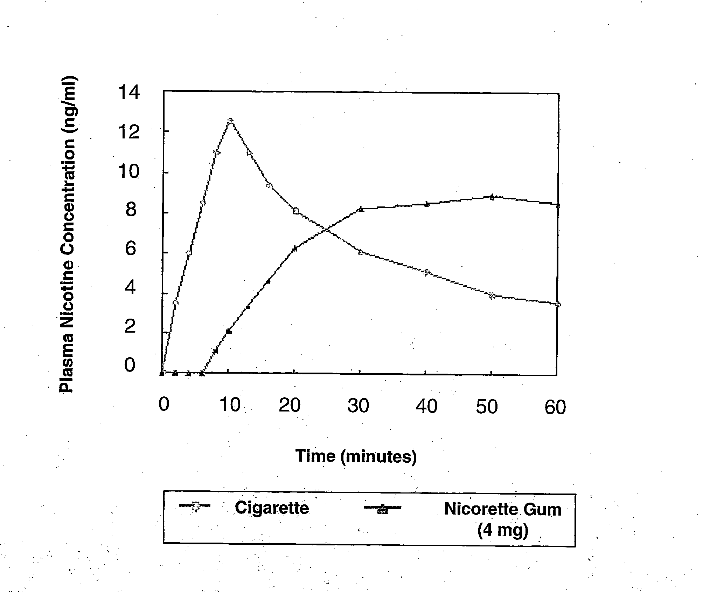

Figure 3 compares the venous blood nicotine plasma profile vs. time of a single dose of the current Nicorette^{®} Gum, extra strength (4 mg), with the corresponding plasma profile when smoking a "light" (low-nicotine) cigarette. One objective with the present invention is to obtain a buccal nicotine formulation providing for a pK profile being closer to the pK profile for a cigarette than is provided using presently known buccal nicotine formulations.

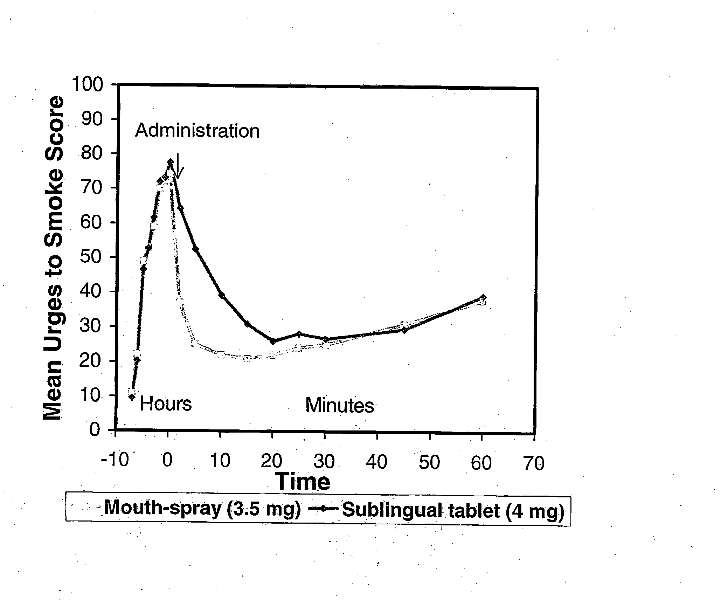

Figure 4 shows the mean score values from 52 smoking volunteers in a randomized open study of the "urge to smoke" (craving), as estimated and recorded on a visual analogue scale (VAS) as a function of time when the same formulations as in Fig. 1 were used. The craving scores were recorded directly after smoking one cigarette and during the abstinence of 7 hours before the administration of the nicotine products. The scores were then recorded more frequently during 1 hour after the administration. The heart rate was also monitored in this study. This figure clearly shows that the present invention provides for a much faster reduction of the urge to smoke score than do present buccal nicotine formulations. For example, about 2 minutes after administration of a formulation according to the present invention the craving score is reduced by 50 %. With Nicorette^{®} Microtab a 50 % decrease in craving score is obtained only more than 10 minutes after administration.

### Detailed Description of the Invention

### Definitions

The terms "tobacco", "tobacco containing material" and similar are herein intended to mean such material for any type of use of tobacco including smoking, snuffing or chewing whereby is used inter alia a cigarette, a cigar, pipe tobacco, snuff and chewing tobacco.

The term "fast reduction of the urge to smoke or use tobacco" is herein intended to mean an initial priming of the subject so as to achieve a reduction of the urge to smoke or use tobacco.

The term "transient" is intended to pertain to a non-permanent change of a biological and/or physiological state, upon which after a certain period of time said state will return to its value or behavior prior to said change.

The term "buccal" and "buccally" are herein intended to pertain to all of or any part of the soft tissue lining of the oral cavity.

The term "liquid of the oral cavity" is herein intended to mean saliva and/or saliva mixed with a quantity of the liquid pharmaceutical formulation.

The term "incidence of administration" is herein intended to mean administration of one or more single doses of the liquid pharmaceutical formulation within the same time frame, said time frame being dependent on the needs of the subject receiving the administration, said time frame extending from a few seconds to around ten minutes.

### The buffering agent

Absorption of nicotine from the oral cavity to the systemic circulation is dependent on the pH of the saliva and the pKa of nicotine, which is about 7 .8. Assuming a pH of the saliva of 6.8 only about 10% of the nicotine in saliva will be in the free base form. Thus, in order to promote absorption of nicotine in a free base form, which is the form predominantly absorbed through the mucosa, the pH of the saliva must be increased. At a pH of 8.8 about 90% of the nicotine in saliva will then be in the free base form.

The liquid pharmaceutical formulation for use according to the invention is a buffered alkaline liquid formulation. This may be achieved by including physiologically acceptable buffering substances.

By buffering thereby increasing the pH of the saliva the uptake of nicotine is changed, e g increased compared to the nicotine uptake when the formulation is not buffered. Also, since the transmucosal uptake of nicotine in the oral cavity according to the invention is faster than for nicotine not being buffered, less nicotine will be swallowed and reach the gastrointestinal (GI) tract. The nicotine that reaches the GI tract will be subjected to first pass metabolism, which reduces the total amount of intact nicotine absorbed additionally reducing the rate of nicotine absorption. This means that the absorption kinetics of nicotine that is not co-administered with a buffer according to the invention will generally be slower and the bioavailability will generally be lower than when administered together with a buffer.

For buffering may be used one or more buffering agents selected from the group consisting of carbonates including bicarbonate or sesquicarbonate, glycinate, phosphate, glycerophosphate or citrate of an alkali metal, such as potassium or sodium, or ammonium, and mixtures thereof.

Further embodiments may use trisodium or tripotassium citrate, and mixtures thereof.

Still further embodiments may comprise different phosphate systems, such as trisodium phosphate, disodium hydrogen phosphate; and tripotassium phosphate, dipotassium hydrogen phosphate, and calcium hydroxide, sodium glycinate; and mixtures thereof.

Alkali metal carbonates, glycinates and phosphates are preferred buffering agents.

The amount of the buffering agent or agents in the liquid pharmaceutical formulation is preferably sufficient in the specific embodiments to raise the pH of the saliva to above 7, and to maintain the pH of the saliva in the oral cavity above 7, e g pH 7 - 11. Otherwise expressed, upon administration to a subject the pH of the liquid of the oral cavity of the
subject is transiently increased by about 0.3 - 4 pH units, preferably by about 0.5 - 2.5 pH units. The amount of buffering agent(s) required to achieve such an increase in pH is readily calculated by a person skilled in the art.

### The active ingredient

According to the invention, the liquid pharmaceutical formulation product comprises nicotine in the free base form to provide a fast transmucosal uptake of the nicotine
in the oral cavity of a subject so as to obtain a reduction of the urge to smoke and/or use tobacco, and/or a rapid "nicotine kick" and/or a "nicotine head rush". Thereby may also be achieved a systemic maintenance level of nicotine.

The nicotine form facilitates the subsequent
uptake of the nicotine from the saliva in the oral cavity into the systemic circulation of the subject.

According to the invention, the uptake of the nicotine through any tissue or mucosa in the oral cavity is improved in relation to the uptake obtained by a liquid nicotine-containing pharmaceutical formulation devoid of alkalizing buffering agents.

The nicotine may act as a stimulant to e.g. obtain a rapid reduction of the urge to smoke or to use tobacco.

### Amount of the nicotine in the liquid pharmaceutical formulation

The nicotine in the alkaline liquid pharmaceutical formulations according to the invention are formulated to provide the subject with a dose to achieve an effect. The effect may be to provide a sense of smoking satisfaction without smoking. Another effect of the administered nicotine may be a reduction of the urge to smoke or use tobacco.

The effect may also be a combination of a reduction of said urge and providing a sense of smoking satisfaction without smoking. The amount of the nicotine should be sufficient to provide such an effect in a subject. This amount may, of course, vary from person to person.

According to the invention, embodiments of the liquid pharmaceutical formulation comprise nicotine in such concentrations that the amount of nicotine delivered at each incidence of administration is about 0.05 - 10 mg calculated as the free base form of nicotine, preferably about 0.25 - 6 mg and most preferably about 0.5-4 mg.

### Release and uptake of nicotine

Presently existing pharmaceutical administration forms for oral administration of nicotine normally provide a slow release and a slow uptake of the nicotine compared to smoking. The slow uptake of the nicotine provides a tₘₐₓ, i e the time-point where the nicotine has its maximum level measured in the plasma of venous blood after a single dose at about 30 - 45 minutes after administration.

The time point for reaching a sense of satisfaction or reduction of urge to smoke or use tobacco after administration is individual, but may in existing pharmaceutical forms for administering nicotine generally be reached after approximately 30 minutes when regarded as coinciding with tₘₐₓ. According to the present invention, such a sense of satisfaction may be reached after a shorter period of time due to a rapid transmucosal uptake in the oral cavity due to the buffering and due to the absence of rate-limiting steps, such as tablet or lozenge melting, tablet or lozenge disintegration and dissolution and chewing gum mastication, followed by drug dissolution.

### The liquid phase

The liquid phase of the present liquid pharmaceutical formulation may comprise water. The liquid phase may also comprise an alcohol, such as ethanol, glycerol, propylene glycol and polyethylene glycol, or mixtures thereof. It may also comprise one or more lipids. Further it may comprise mixtures of the above ingredients.

### Other additives to the liquid pharmaceutical formulation

Other additives may be added optionally to the liquid pharmaceutical formulation.

Optional additives comprise one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethylal alcohol, cetylpyridinium chloride; and chelating agents, such as EDTA; and galates, such as propyl galate.

Further optional additives comprise one or more additives selected from the group consisting of:
- enhancers, such as azone;
- vitamins, such as vitamins C and E;
- minerals, such as fluorides, especially sodium fluoride, sodium monofluoro phosphate and stannous fluoride;
- anti-odours, such as zinc and cyclodextrins;
- propellants, such as 1,1,2,2-tetrafluoroethane (HFC-134a), optionally being liquefied, and 1,1,1,2,3,3,3-heptafluororpropane (HFC-227), optionally being liquefied;
- sweeteners including one or more synthetic sweetening agents and/or natural sugars, such as those selected from the groups consisting of artificial sweeteners e g saccharin and its sodium and calcium salts, aspartame, acesulfame and its potassium salt, thaumatin and glycyrrhizin;
- polyhydric alcohols such as sorbitol, xylitol, mannitol and glycerol;
- monosaccharides including glucose (also called dextrose), fructose (also called laevulose) and galactose;
- disaccharides including saccharose (also called sucrose), lactose (also called milk sugar) and maltose (also called malt sugar);
- mixtures of sugars including liquid glucose syrup e g starch hydrolysates containing a mixture of chiefly dextrose, maltose, dextrins and water, invert sugar syrup e g sucrose inverted by invertase containing a mixture of dextrose, laevulose and water, high sugar content syrups such as treacle, honey and malt extract; and mixtures thereof;
- flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary;
- natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews;
- synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews;
- and mixtures thereof.

### Surface active agents

One or more of the compounds of the liquid pharmaceutical formulation may be solubilized in one or more surface active agents and/or emulsifiers, such as nonionic, cationic, anionic or zwitterionic surfactants, including amphiphilic block copolymers, or mixtures thereof.

Specifically one or more of the compounds of the liquid pharmaceutical formulation may be solubilized in one or more surface-active agents selected from nonionic surface-active agents including poloxamers, e g:
- poly(oxypropylene)-poly(oxyethylene) block copolymers, polyoxyethylene alkyl ethers, polyoxyethylene castor oil derivatives, polyoxyethylene sorbitan fatty acid esters, mono- and diglycerides and esters thereof, polyoxyethylene stearates, polyglycerolesters of fatty acids (including polyglycerolpolyricinoleic acid (PGPR)), and sorbitan fatty acid esters,
- cationic surface-active agents including secondary, quaternary and tertianary ammonium compounds and cationic phospholipids,
- anionic surface-active agents including fatty acid salts, lactylates, especially sodium and/or calcium stearoyllactylate, alkyl sulphates, alkyl sulphonates, latanol, and anionic phospholipids, such as phosphatidylserine,
- zwitterionic surface-active agents including zwitterionic phospholipids, such as phosphatidylcholine and phosphatidylethanolamine,
- or mixtures thereof,
- preferably surface-active agents or mixtures thereof being nonionic.

### Method for delivering nicotine in any form to a subject

According to the disclosure, a method for delivering nicotine to a subject comprises the steps of:
a) administering to a subject a liquid pharmaceutical formulation product containing nicotine according to the invention into the oral cavity of the subject, and
b) allowing the nicotine in the liquid pharmaceutical formulation to be mixed with the saliva in the oral cavity and absorbed into the blood plasma of the subject essentially by buccal uptake.

One embodiment results in a tₘₐₓ of nicotine in venous blood of the subject after about 3 - 30 minutes.

One further embodiment results in a tₘₐₓ of nicotine in venous blood of the subject after about 3 - 20 minutes.

In still one further embodiment, said nicotine is absorbed resulting in a tₘₐₓ of nicotine in venous blood of the subject after about 3 - 15 minutes.

### Method for obtaining reduction of the urge to smoke or use tobacco

A method for obtaining reduction of the urge to smoke or use tobacco-containing material and/or for providing a sense of smoking satisfaction without smoking according to the disclosure comprises the steps of:
a) replacing at least partly the tobacco containing material with a liquid pharmaceutical formulation as claimed,
b) administering to a subject a liquid pharmaceutical formulation containing nicotine -as claimed into the oral cavity of the subject, and
c) allowing the nicotine in the liquid pharmaceutical formulation to be absorbed by the subject essentially by buccal uptake.

The administration to the oral cavity takes place by spraying, dropping or pipetting, preferably by spraying, most preferably by spraying under the tongue. The administration is intended for the oral cavity, not for e g the lungs or the upper respiratory tract.

In one embodiment said nicotine results in a tₘₐₓ of nicotine in venous blood of the subject after about 3 - 30 minutes.

In one further embodiment said nicotine results in a tₘₐₓ of nicotine in venous blood of the subject after about 3 - 20 minutes.

In still one further embodiment said nicotine results in a tₘₐₓ of nicotine in venous blood of the subject after about 3 - 15 minutes.

Even further embodiments of the method for delivering nicotine to a subject may comprise the steps of combining at least one other method for obtaining reduction of the urge to smoke or use of tobacco.

The liquid pharmaceutical formulation may be used for obtaining a quick and/or sustained and/or complete reduction of the urge to smoke or use tobacco and/or for providing a sense of smoking satisfaction without smoking as further discussed below.

The fast relief provides the subject with a sense of rapid smoking satisfaction without smoking.

One embodiment reduces the urge to smoke or use of tobacco by reaching a tₘₐₓ of nicotine in venous blood of the subject after about 3 - 30 minutes by the use of a liquid pharmaceutical formulation according to the invention.

One further embodiment reduces the urge to smoke or use tobacco by reaching a tₘₐₓ of nicotine in venous blood of the subject after about 3 - 20 minutes by the use of a liquid pharmaceutical formulation according to the invention.

Still one further embodiment reduces the urge to smoke or use tobacco-by reaching a tₘₐₓ of nicotine in venous blood of the subject after about 3 - 15 minutes by the use of a liquid pharmaceutical formulation according to the invention.

### Cessation of the urge to smoke or use tobacco

For some of the users, it may be a goal to terminate the usage of nicotine completely, due to several reasons e g health, economical, social or behavioral. This may be achieved by further decreasing the delivered amount of nicotine in any form gradually over time. In specific embodiments of the invention, the method described above for obtaining craving relief may further comprise the steps of decreasing the amount of nicotine in the liquid pharmaceutical formulation gradually over time, and/or the steps of reducing the incidence of administration of the liquid pharmaceutical formulation gradually over time, and/or the steps of reducing the dosage size of the liquid pharmaceutical formulation gradually over time, so as to achieve a relief of tobacco craving and/or to achieve a sense of smoking satisfaction. This method results in a weaning process gradually over time.

Different types of smokers reach the sense of reduced craving at different plasma levels of nicotine. This may, of course, affect the individual types of programs for administering a liquid pharmaceutical formulation according to the invention. Different types of smokers include e g peak seekers or smokers that crave for a plasma level of nicotine constantly being above the level below which withdrawal symptoms occur.

One strategy may be to lower the frequency of administering the liquid pharmaceutical formulation. Other embodiments include varying the dose of the nicotine in said liquid pharmaceutical formulation as well as the combination of these two embodiments.

### Systems for delivering nicotine and for obtaining craving relief

According to the invention there is a system for delivering nicotine to a subject. Such a system comprises a liquid pharmaceutical formulation according to the invention and at least one other means for obtaining reduction of the urge to smoke.

Another system according to the invention may be a system for obtaining reduction of the urge to smoke or use tobacco and/or for providing a sense of smoking satisfaction without smoking. Such a system comprises a liquid pharmaceutical formulation according to the invention and at least one other method for obtaining reduction of the urge to smoke or use tobacco. Other methods may be a concomitant or concurrent method selected from the group consisting of administration through chewing gums, nasal sprays, transdermal patches, inhaling devices, lozenges, tablets and parenteral methods, subcutaneous methods, intravenous methods, rectal methods, vaginal methods and transmucosal methods; or use of tobacco.

In a specific embodiment, the at least one other method comprises administration of nicotine.

### Use of the liquid pharmaceutical formulation

The use of the liquid pharmaceutical formulation according to the invention is for obtaining a fast and/or complete reduction of the urge to smoke and use tobacco or for providing a sense of smoking without smoking as described above.

The dose of the nicotine is chosen to give the subject an individual sensory perception and satisfaction with an effect of the nicotine. The use of the liquid pharmaceutical formulation may also be a sole use according to the invention or a combination with other means or methods known in the field of drug abuse. Specifically, the present invention may be used in combination with other means as described above in the methods in the paragraphs above.

The use may give a quick reduction of the urge to smoke or use tobacco whereby is reached a tₘₐₓ of nicotine in venous blood after about 3 - 20 minutes.

In a specific embodiment, the use of the liquid pharmaceutical formulation according to the invention will reduce the urge to smoke or use tobacco by reaching a tₘₐₓ of nicotine in venous blood of the subject after about 3 - 15 minutes.

According to the invention, a use of a liquid pharmaceutical formulation according to the invention is for delivering nicotine to a subject.

In one embodiment, the delivering of nicotine results in a tₘₐₓ of nicotine in venous blood of the subject after about 3 - 30 minutes.

In another embodiment, the delivering of nicotine results in a tₘₐₓ of nicotine in venous blood of the subject after about 3 - 20 minutes.

In still another embodiment, the delivering of nicotine results in a tₘₐₓ of nicotine in venous blood of the subject after about 3 - 15 minutes.

As readily shown and concluded from the figures, e g Figure 4, the shorter the tₘₐₓ the faster the relief of the craving, i e of the urge to smoke.

### Examples on embodiments and manufacturing of the liquid pharmaceutical formulation

The below examples are non-limiting and for illustrating the present invention. Alternatives and variations of the below examples within the scope of the present invention as per the below claims may be carried out by a person skilled in the art. Ingredients as per the below examples may be exchanged for equivalent ingredients, preferably as per above. The formulations according to Examples 2 and 3 were made for comparative purposes as seen from Figure 2.

### Example 1

Manufacturing of a 1000 ml formulation with 10 mg nicotine/ml and around pH8.5.

### Mixture 1

To a beaker containing 800 ml water of 90°C was added 0.7 g methyl para-hydroxybenzoate, acting as preservative, and 0.3 g propyl para-hydroxybenzoate, acting as preservative. The additives were dissolved during stirring for about 10 minutes. Then was added 10.45 g sodium dihydrogen phosphate, acting as buffering agent, and 0.5 g EDTA, acting as chelating agent, to the solution, which was stirred for about 5 minutes. Then the solution was cooled to 30°C during stirring.

### Mixture 2

To a beaker containing 15.9 g ethanol of room temperature, acting as solvent, was added 0.045 g peppermint oil, acting as flavoring agent. The liquid was mixed 30 for 2 minutes.

### Final mixture

Mixture 2 was added during stirring to a beaker containing 150 ml water. Gently 10 g nicotine (base) was added to the beaker. Then Mixture 1 was added to the beaker and stirred for 5 minutes. The pH of the Final mixture was checked and adjusted to about pH 8.5 with sodium hydroxide (20%) and to volume with water.

### Comparative Example 2

Manufacturing of a 1000 ml formulation with 10 mg nicotine/ml and around pH 7.0.

This Example 2 differs from Example 1 only for pH. The formulation according to Example 2 contains a non-alkalizing buffering agent. This formulation was for use as a comparison in Figure 2.

### Mixture 1

To a beaker containing 800 ml water of 90°C was added 0.7 g methyl para-hydroxybenzoate, acting as preservative, and 0.3 g propyl para-hydroxybenzoate, acting as preservative. The additives were dissolved during stirring for about 10 minutes. Then was added 10.45 g sodium dihydrogen phosphate, acting as buffering agent, and 0.5 g EDTA, acting as chelating agent, to the solution, which was stirred for about 5 minutes. Then the solution was cooled to 30°C during stirring.

### Mixture 2

To a beaker containing 15.9 g ethanol of room temperature, acting as solvent, was added 0.045 g peppermint oil, acting as flavoring agent. The liquid was mixed for 2 minutes.

### Final mixture

Mixture 2 was added during stirring to a beaker containing 150 ml water. Gently 10 g nicotine (base) was added to the beaker. TI1 en Mixture 1 was added to the beaker and stirred for 5 minutes. The pH of the Final mixture was checked and adjusted to about p.H 7 .0 with hydrochloric acid and to volume with water.

### Comparative Example 3

Manufacturing of a 1000 ml formulation with 10 mg nicotine/ml and around pH 6.0.

This Example 3 differs from Example 1 only for pH. The formulation according to Example 3 contains a non-alkalizing buffering agent. This formulation was for use as a comparison in Figure 2.

### Mixture 1

To a beaker containing 800 ml water of 90°C was added 0.7 g methyl para-hydroxybenzoate, acting as preservative, and 0.3 g propyl para-hydroxybenzoate, acting as preservative. The additives were dissolved during stirring for about 10 minutes. Then was added 10.45 g sodium dihydrogen phosphate, acting as buffering agent, and 0.5 g EDTA, acting as chelating agent, to the solution, which was stirred for about 5 minutes. Then the solution was cooled to 30°C during stirring.

### Mixture 2

To a beaker containing 15.9 g ethanol of room temperature, acting as solvent, was added 0.045 g peppermint oil, acting as flavoring agent. The liquid was mixed for 2 minutes.

### Final mixture

Mixture 2 was added during stirring to a beaker containing 150 ml water. Gently 10 g nicotine (base) was added to the beaker. Then Mixture 1 was added to the beaker and stirred for 5 minutes. The pH of the Final mixture was checked and adjusted to about pH 6.0 with hydrochloric acid and to volume with water.

### Example 4

Manufacturing of a 1000 ml formulation with 17 .5 mg nicotine/ml and around pH 9.0.

### Mixture 1

To a beaker containing 600 ml water of room temperature was added 12.0 g Synperonic^{®} PE/F27, being a poloxamer acting as non-ionic surface active agent. The additive was dissolved during stirring for about 20 minutes. Then was added 0.5 g EDTA, acting as chelating agent, and 0.4 g sodium saccharin, acting as sweetener, to the liquid which was stirred until all ingredients were dissolved. Then was added 16.8 g sodium hydrogen carbonate, acting as buffering agent, and the solution was stirred until a clear solution was obtained.

### Mixture 2

To a beaker containing 250.0 g ethanol of room temperature, acting as solvent, was added 0.7 g methyl para-hydroxybenzoate acting as preservative, and 0.3 g propyl para-hydroxybenzoate acting as preservative. The liquid was mixed until the ingredients were dissolved. Then was added 5.0 g peppermint oil, acting as flavoring agent, and 1.5 g aroma agent. The liquid was mixed until a clear solution was obtained.

### Final mixture

Mixture 2 was gently added to Mixture 1 during stirring for about 1 minute. Then was added 17.5 g nicotine (base) and the liquid was stirred for about 2 minutes. The pH of the Final mixture was checked and adjusted to around pH 9 .0 with hydrochloric acid. The Final mixture was transferred to a 1000 ml volumetric flask and adjusted to 1000 ml volume by water. Finally the pH of the solution was checked to remain at around pH 9.0.

### Example 5

Manufacturing of a 1000 ml formulation with 14.3 mg nicotine/ml and around pH 9.0.

### Mixture 1

To a beaker containing 600 ml water of room temperature was added 20.0 g Synperonic® PEIF27 being a poloxamer, acting as non-ionic surface active agent. The additive was dissolved during stirring for about 20 minutes. Then was added 2.0 g Acesulfame K, acting as sweetener, to the liquid which was stirred until all ingredients were dissolved. Then was added 20.0 g sodium hydrogen carbonate, acting as buffering agent, and the liquid was stirred until a clear solution was obtained.

### Mixture 2

To a beaker containing 95.0 g ethanol of room temperature, acting as solvent, was added 3.5 g peppermint oil, acting as flavoring agent, and 1.0 g aroma agent. The liquid was mixed until a clear solution was obtained.

### Final mixture

Mixture 2 was gently added to Mixture 1 during stirring for about 1 minute. Then was added 14.3 g nicotine (base) and the liquid was stirred for about 2 minutes. The pH of the Final mixture was checked and adjusted to around pH 9 .0 with hydrochloric acid. The Final mixture was transferred to a 1000 ml volumetric flask and adjusted to 1000 ml volume by water. Finally the pH of the solution was checked to remain at around pH 9.0.

The formulation according to Example 5 is a preferred composition.

### Example 6

Manufacturing of a 1000 ml formulation with 14.3 mg nicotine/ml and around pH 9.0.

### Mixture 1

To a beaker containing 600 ml water of room temperature was added 20.0 g Synperonic® PE/F27 being a poloxamer, acting as non-ionic surface active agent. 15 The additive was dissolved during stirring for about 20 minutes. Then was added 0.2 g benzalkonium chloride, acting as preservative, and 2.0 g Acesulfame K, acting as sweetener, to the liquid which was stirred until all ingredients were dissolved. Then was added 20.0 g sodium hydrogen carbonate, acting as buffering agent, and the liquid was stirred until a clear solution was obtained.

### Mixture 2

To a beaker containing 95.0 g ethanol of room temperature, acting as solvent, was added 3.5 g peppermint oil, acting as flavoring agent, and 1.0 g aroma agent. The liquid was mixed until a clear solution was obtained.

### Final mixture

Mixture 2 was gently added to Mixture 1 during stirring for about 1 minute. Then was added 14.3 g nicotine (base) and the liquid was stirred for about 2 minutes. The pH of the Final mixture was checked and adjusted to around pH 9.0 with hydrochloric acid. The Final mixture was transferred to a 1000 ml volumetric flask and adjusted to 1000 ml volume by water. Finally the pH of the solution was checked to remain at around pH 9.0.

The formulation according to Example 6 is a another preferred composition.

### Comparative Example 7

Manufacturing of a 1000 ml formulation with 14.3 mg nicotine/ml and around pH 9.0.

### Mixture 1

To a beaker containing 600 ml water of room temperature was added 20.0 g Synperonic® PE/F27 being a poloxamer, acting as non-ionic surface active agent. The additive was dissolved during stirring for about 20 minutes. Then was added 0.5 g EDTA, acting as chelating agent, and 2.0 g Acesulfame K, acting as sweetener, to the liquid which was stirred until all ingredients were dissolved. Then 10 was added 20.0 g sodium hydrogen carbonate, acting as buffering agent, and the liquid was stirred until a clear solution was obtained.

### Mixture 2

To a beaker containing 95.0 g ethanol of room temperature, acting as solvent, was added 0.7 g methyl para-hydroxybenzoate acting as preservative, and 0.3 g propyl para-hydroxybenzoate acting as preservative. The liquid was mixed until the ingredients were dissolved. Then was added 3.5 g peppermint oil, acting as flavoring agent, and 1.0 g aroma agent. The liquid was mixed until a clear solution was obtained.

### Final mixture

Mixture 2 was gently added to Mixture 1 during stirring for about 1 minute. Then was added about 2 ml sodium hydroxide (50%) and 4 g nicotine bitartrate. The pH of the Final mixture was not allowed to decrease below pH 8 during the addition of the nicotine bitartrate. The preceding procedure with adding of sodium hydroxide and nicotine bitartrate was repeated until totally 40.7 g nicotine bitartrate 25 was added. The pH of the Final mixture was adjusted to around pH 9.0. The Final mixture was transferred to a 1000 ml volumetric flask and adjusted to 1000 ml volume by addition of water. Finally the pH of the solution was checked to remain at around pH 9.0.

### Comparative Example 8

Manufacturing of a 1000 ml formulation with 17.5 mg nicotine/ml and pH 10.94.

To a beaker containing 950 ml water of room temperature was added 17.5 g nicotine (base) during stirring for about 5 minutes. The volume was adjusted to 1000 ml volume by addition of water. Finally the pH was checked.

### Example 9

Manufacturing of a 1000 ml formulation with 17.5 mg nicotine/ml and pH 11.55.

To a beaker containing 950 ml water of room temperature was added 35 g sodium carbonate anhydrous during stirring until complete dissolution. Then 17.5 g nicotine-(base) was added during stirring for about 5 minutes. The volume was adjusted to 1000 ml volume by addition of water. Finally the pH was checked.

### Example 10

Manufacturing of a 1000 ml formulation with 15.65 mg nicotine/ml and pH 11.79.

To a beaker containing 950 ml water of room temperature was added 158 g glycine sodium salt during stirring until complete dissolution. Then 15.65 g nicotine (base) was added during stirring for about 5 minutes. The volume was adjusted to 1000 ml volume by addition of water. Finally the pH was checked.

### Example 11

### Buffer capacity determinations

**Method:** 10.0 ml of the respective below solutions was titrated with 0.1 M HCl to pH 7.0. The amount of 0.1 M HCl needed to decrease pH from 9.0 to 8.0 was determined.

**Definitions:**
(1) Sodium hydrogen carbonate (NaHC0₃). Mw: 84.0
(2) Disodium phosphate dodecahydrate (Na₂HP0₄,12H20) Mw: 358.1

| **Ingredient\Batch** | DKN0293 | DKN0294 | DKN0295 | DKN0296 | DKN0290 | DKN0291 |
|---|---|---|---|---|---|---|
| Nicotine (mg/ml) | | | 10.0 | | 10.0 | 10.0 |
| NaHCO₃ (mg/ml) | 16.8 | | 16.8 | 8.4 | | |
| Na₂HPO₄, 12H₂O (mg/ml) | | 71.6 | | 35.8 | 71.6 | |
| Purified water ad | 1 ml | 1 ml | 1 ml | 1 ml | 1 ml | 1 ml |
| Buffer Capacity PH=9.0-8.0 (mekv/l) | 26.5 | 9.5 | 50 | 15.8 | 40 | 29 |

All solutions were adjusted to a pH of 9.0 when needed. A higher pH may cause irritation and corrosion, which might be harmful to the tissue of the oral cavity.

| | |
|---|---|
| 16.8 mg/ml of | NaHCO₃ corresponds to 0.2 M. |
| 71.6 mg/ml of | Na₂HPO₄,12H₂O corresponds to 0.2 M. |
| 8.4 mg/ml of | NaHCO₃ corresponds to 0.1 M. |
| 35.8 mg/ml of | Na₂HPO₄,12H₂O corresponds to 0.1 M |

Nicotine base has an alkalizing effect, but has too weak a buffering capacity on its own. The buffering capacity of the formulation is significantly and sufficiently increased when a buffering agent is added.

The above data clearly show that the present formulations have a good buffering capacity, providing for the desired rapid transmucosal uptake of nicotine.

A liquid pharmaceutical formulation according to the present invention may be administered using suitable devices being available on the market, e g spray devices.

### Analysis of nicotine

The analysis of nicotine uptake and of the effect of the invention may be done according to standard procedures known in the art, e g using a bioanalysis for the determination of nicotine in the plasma of a subject.

### Effects of the invention

Comparative tests were conducted as described above under Legend of figures.

Figure 1 shows that with a liquid pharmaceutical formulation according to the present invention the venous blood plasma level of nicotine ascends signifycantly more rapidly than with Nicorette Microtab®. Nicorette Microtab® has the same pharmacokinetic profile as, i e is pharmacologically equivalent with, Nicorette Gum® and all other nicotine chewing gums currently on the market. Nicotine chewing gums presently represent around half of the world sales of medicinal nicotine-containing products for smoking cessation and similar indications.

Figure 2 shows that the higher the pH of a liquid pharmaceutical formulation according to the present invention the faster the absorption kinetics and the higher the plasma concentration of nicotine.

Figures 3 and 4 further show that a formulation according to the present invention provides for a fast craving relief manifested through a significantly faster reduction in the urge to smoke compared to known oral nicotine formulations.

### Use for therapy, treatment and manufacturing

The liquid pharmaceutical formulation product according to the invention may be used in therapy. Said therapy may be a treatment of a disease or medical indication selected from the group consisting of reduction in use of tobacco, cessation of use of tobacco, other use of tobacco, temporary abstinence from abstaining from use of tobacco.

Nicotine may be used for the manufacturing of a liquid pharmaceutical formulation according to the invention for the treatment of a disease or medical indication selected from the group consisting of reduction in use of tobacco, cessation of use of tobacco, other use of tobacco, temporary abstinence from abstaining from using tobacco.

## Claims

1. A pharmaceutical formulation for use in the treatment of addiction to tobacco or nicotine comprising nicotine and **characterised in that**:
(i) the nicotine is present as a free nicotine base;
(ii) the pharmaceutical formulation is a buffered alkaline liquid formulation for administration to the oral cavity of a subject by spraying, dropping or pipetting; and in that
(iii) the nicotine is co-administered with a buffering agent.

2. A pharmaceutical formulation for use as claimed in Claim 1 wherein the formulation is for administration to the oral cavity of a subject by spraying.

3. A pharmaceutical formulation for use according to Claim 1 or 2 **characterised in that** one or more buffering agents is selected from the group consisting of a carbonate, such as mono-carbonate, bicarbonate or sesquicarbonate; glycinate, phosphate, glycerophosphate, acetate, gluconate or citrate of an alkali metal, such as potassium or sodium, or of ammonium, and mixtures thereof.

4. A pharmaceutical formulation for use according to any of Claims 1 - 3, wherein the formulation has a liquid phase which comprises water.

5. A pharmaceutical formulation for use according to any of Claim 1 - 4, wherein the liquid phase comprises an alcohol, such as ethanol, glycerol, propylene glycol and polyethylene glycol, or mixtures thereof.

6. A pharmaceutical formulation for use according to any of Claims 1 - 5, wherein the liquid phase comprises one or more lipids.

7. A pharmaceutical formulation for use according to any of Claims 1 - 6, wherein the liquid phase comprises water and/or an alcohol, such as ethanol, glycerol, propylene glycol and polyethylene glycol, and/or one or more lipids or mixtures thereof.

8. A pharmaceutical formulation for use according to any of Claims 1 - 7, **characterised in that** it further comprises one or more flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary;
natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews;
synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews;
and mixtures thereof.

9. A pharmaceutical formulation for use according to any of Claims 1 - 8, **characterised in that** it further comprises one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethylal alcohol, cetylpyridinium chloride; and chelating agents, such as EDTA; and galates, such as propyl galate.

10. A pharmaceutical formulation for use according to any of Claims 1 - 9, **characterised in that** it further comprises one or more additives selected from the group consisting of:
thickening agents, enhancers, vitamins, minerals, anti-odours, propellants, sweeteners including one or more synthetic sweetening agents and/or natural sugars, such as those selected from the groups consisting of artificial sweeteners, polyhydric alcohols, disaccharides and mixtures thereof.

11. A pharmaceutical formulation for use according to any of Claims 1 - 10, **characterised in that** one or more of the compounds of the pharmaceutical formulation is/are solubilised in one or more surface active agents and/or emulsifiers, such as nonionic, cationic, anionic or zwitterionic surfactants, including amphiphilic block copolymers, or mixtures thereof.

12. A pharmaceutical formulation for use according to any of Claims 1 - 11, **characterised in that** it comprises free nicotine base, sodium hydrogen carbonate acting as buffering agent, water acting as solvent, ethanol acting as co-solvent, a poloxamer acting as surface active agent, EDTA acting as chelating agent, Acesulfame K acting as sweetener, optionally one or more preservatives, and optionally one or more flavoring or aroma agents.

13. A pharmaceutical formulation for use according to any of Claims 1 -12 whereby the treatment provides a quick and/or complete reduction of the urge to smoke or use tobacco and/or for providing a sense of smoking satisfaction without smoking.

14. A liquid pharmaceutical formulation for use as claimed in any one of Claims 1 - 13 wherein the formulation comprises nicotine in such concentration that the amount of nicotine delivered at each incidence of administration is 0.05 - 10 mg.

15. A liquid pharmaceutical formulation for use as claimed in Claim 14 wherein the amount of nicotine delivered at each incidence of administration is 0.25 - 6 mg.

16. A liquid pharmaceutical formulation for use as claimed in Claim 14 wherein the amount of nicotine delivered at each incidence of administration is 0.5 - 4 mg.

## Patentansprüche

1. Pharmazeutische Formulierung zur Verwendung bei der Behandlung von Tabak- oder Nikotinabhängigkeit, die Nikotin umfasst und **dadurch gekennzeichnet** ist, dass:
(i) das Nikotin als eine freie Base präsent ist;
(ii) die pharmazeutische Formulierung eine gepufferte Alkali-Flüssigkeit-Formulierung zur Verabreichung in die Mundhöhle eines Subjektes durch Sprühen, Tropfen oder Pipettieren ist; und
(iii) das Nikotin gemeinsam mit einem Puffermittel verabreicht wird.

2. Pharmazeutische Formulierung zur Verwendung nach Anspruch 1, wobei die Formulierung zur Verabreichung in die Mundhöhle eines Subjektes durch Sprühen ausgebildet ist.

3. Pharmazeutische Formulierung zur Verwendung nach Anspruch 1 oder 2, **dadurch gekennzeichnet, dass** ein oder mehrere Puffermittel ausgewählt sind aus der Gruppe, bestehend aus einem Carbonat, wie beispielsweise Mono-Carbonat, Bi-Carbonat oder Sesqui-Carbonat; Glycinat, Phosphat, Glycerophosphat, Acetat, Gluconat oder Citrat eines Alkalimetalls, wie beispielsweise Kalium oder Natrium, oder von Ammonium, und Mischungen davon.

4. Pharmazeutische Formulierung zur Verwendung nach einem der Ansprüche 1 bis 3, wobei die Formulierung eine flüssige Phase aufweist, die Wasser umfasst.

5. Pharmazeutische Formulierung zur Verwendung nach einem der Ansprüche 1 bis 4, wobei die flüssige Phase einen Alkohol umfasst, wie beispielsweise Ethanol, Glyzerin, Propylenglykol und Polyethylenglykol oder Mischungen davon.

6. Pharmazeutische Formulierung zur Verwendung nach einem der Ansprüche 1 bis 5, wobei die flüssige Phase ein oder mehrere Lipide umfasst.

7. Pharmazeutische Formulierung zur Verwendung nach einem der Ansprüche 1 bis 6, wobei die flüssige Phase Wasser und/oder einen Alkohol, wie beispielsweise Ethanol, Glyzerin, Propylenglykol und Polyethylenglykol, und/oder ein oder mehrere Lipide oder Mischungen davon umfasst.

8. Pharmazeutische Formulierung zur Verwendung nach einem der Ansprüche 1 bis 7, **dadurch gekennzeichnet, dass** sie ferner ein oder mehrere Geschmacksmittel oder Aromatisierungsmittel umfasst, wie beispielsweise jene, die ausgewählt sind aus der Gruppe, bestehend aus ätherischen Ölen, erhalten durch Destillationen, Lösungsmittelextraktionen oder Kaltpressungen von frischen oder getrockneten Blüten, Knospen, Blättern, Stängeln, Früchten, Samen, Schalen, Rinden oder Wurzeln, zum Beispiel Öl von Pfefferminze, grüner Minze, Eukalyptus, Wintergrün, Niauli, Nelke, Kardamom, Zimt, Bittermandel, Koriander, Kümmel, Ingwer, Wacholder, Orange, Bitterorange, Zitrone, Grapefruit, Mandarine, Bergamotte, Thymian, Fenchel und Rosmarin;
natürlichen Aromen und Aromastoffen, umfassend entweder verdünnte Lösungen von ätherischen Ölen oder Konzentraten von Geschmackskomponenten mit natürlichem Ursprung von zum Beispiel Früchten, Beeren, Nüssen, Gewürzen, Minzen, Tabak, Kakao, Kaffee, Tee, Vanille, Süßholzwurzeln, Karamell, Toffee, Honig, Wein, Likören und Gebräuen;
synthetischen Aromen und Aromastoffen, die aus Mischungen von Chemikalien bestehen, umfassend Kohlenwasserstoffe, Alkohole, Aldehyde, Ester, Ketone, Ether und Oxide, die gemischt sind, um mit dem natürlichen Aroma von zum Beispiel Früchten, Beeren, Nüssen, Gewürzen, Minzen, Tabak, Kakao, Kaffee, Tee, Vanille, Süßholzwurzeln, Karamell, Toffee, Honig, Wein, Likören und Gebräuen übereinzustimmen;
und Mischungen davon.

9. Pharmazeutische Formulierung zur Verwendung nach einem der Ansprüche 1 bis 8, **dadurch gekennzeichnet, dass** sie ferner ein oder mehrere stabilisierende Additive umfasst, wie beispielsweise jene, die ausgewählt sind aus der Gruppe, bestehend aus Antioxidantien, einschließlich Vitamin E, das heißt Tocopherole, Vitamin C, das heißt Ascorbinsäure und ihre Salze, Natriumpyrosulfit, Butylhydroxytoluol, butyliertes Hydroxyanisol; und Konservierungsstoffen, einschließlich Parabene, Benzalkoniumchlorid, Chlorbutanol, Benzylalkohol, Beta-Phenylethylalalkohol, Cetylpyridiniumchlorid; und Chelatbildnern, wie beispielsweise EDTA; und Galaten, wie beispielsweise Propylgalat.

10. Pharmazeutische Formulierung zur Verwendung nach einem der Ansprüche 1 bis 9, **dadurch gekennzeichnet, dass** sie ferner ein oder mehrere Additive umfasst, die ausgewählt sind aus der Gruppe, bestehend aus Verdickungsmitteln, Verstärkern, Vitaminen, Mineralien, Anti-Geruchsmitteln, Treibmitteln, Süßstoffen, einschließlich eines oder mehrerer synthetischer Süßungsmittel und/oder natürlicher Zucker, wie beispielsweise jene, die ausgewählt sind aus der Gruppe, bestehend aus künstlichen Süßstoffen, mehrwertigen Alkoholen, Disacchariden und Mischungen davon.

11. Pharmazeutische Formulierung zur Verwendung nach einem der Ansprüche 1 bis 10, **dadurch gekennzeichnet, dass** eine oder mehrere der Verbindungen der pharmazeutischen Formulierung löslich gemacht ist/sind in einem oder mehreren oberflächenaktiven Mitteln und/oder Emulgatoren, wie beispielsweise nicht-ionischen, kationischen, anionischen oder zwitterionischen Tensiden, einschließlich amphiphiler Block-Copolymere, oder Mischungen davon.

12. Pharmazeutische Formulierung zur Verwendung nach einem der Ansprüche 1 bis 11, **dadurch gekennzeichnet, dass** sie eine freie Nikotin-Base, Natriumhydrogencarbonat, das als Puffermittel wirkt, Wasser, das als Lösungsmittel wirkt, Ethanol, das als Co-Lösungsmittel wirkt, ein Poloxamer, das als oberflächenaktives Mittel wirkt, EDTA, das als Chelatbildner wirkt, Acesulfam K, das als Süßungsmittel wirkt, gegebenenfalls ein oder mehrere Konservierungsmittel und gegebenenfalls ein oder mehrere Geschmacksmittel oder Aromastoffe umfasst.

13. Pharmazeutische Formulierung zur Verwendung nach einem der Ansprüche 1 bis 12, wobei die Behandlung eine schnelle und/oder vollständige Reduktion des Verlangens, zu rauchen oder Tabak zu verwenden, bereitstellt und/oder zum Bereitstellen eines Gefühls der Rauchbefriedigung ohne Rauchen.

14. Flüssige pharmazeutische Formulierung zur Verwendung nach einem der Ansprüche 1 bis 13, wobei die Formulierung Nikotin in einer Konzentration umfasst, dass die Menge an Nikotin, die bei jedem Ereignis einer Verabreichung verabreicht wird, 0,05 bis 10 mg beträgt.

15. Flüssige pharmazeutische Formulierung zur Verwendung nach Anspruch 14, wobei die Menge an Nikotin, die bei jedem Ereignis einer Verabreichung verabreicht wird, 0,25 bis 6 mg beträgt.

16. Flüssige pharmazeutische Formulierung zur Verwendung nach Anspruch 14, wobei die Menge an Nikotin, die bei jedem Ereignis einer Verabreichung verabreicht wird, 0,5 bis 4 mg beträgt.

## Revendications

1. Formulation pharmaceutique pour son utilisation dans le traitement d'une addiction au tabac ou à la nicotine comprenant de la nicotine et **caractérisée en ce que** :
(i) la nicotine est présente sous forme de base de nicotine libre ;
(ii) la formulation pharmaceutique est une formulation liquide alcaline tamponnée pour l'administration au niveau de la cavité buccale d'un sujet par pulvérisation, goutte-à-goutte ou pipetage ; et en ce que
(iii) la nicotine est co-administrée avec un agent tampon.

2. Formulation pharmaceutique pour son utilisation selon la revendication 1, dans laquelle la formulation est destinée à une administration dans la cavité buccale d'un sujet par pulvérisation.

3. Formulation pharmaceutique pour son utilisation selon la revendication 1 ou 2, **caractérisée en ce qu'**un ou plusieurs agents tampons sont choisis dans le groupe constitué par un carbonate tel que le monocarbonate, le bicarbonate ou le sesquicarbonate ; un glycinate, un phosphate, un glycérophosphate, un acétate, un gluconate ou un citrate d'un métal alcalin tel que le potassium ou le sodium, ou d'ammonium, et des mélanges de ceux-ci.

4. Formulation pharmaceutique pour son utilisation selon l'une quelconque des revendications 1 à 3, dans laquelle la formulation a une phase liquide qui comprend de l'eau.

5. Formulation pharmaceutique pour son utilisation selon l'une quelconque des revendications 1 à 4, dans laquelle la phase liquide comprend un alcool tel que l'éthanol, le glycérol, le propylène glycol et le polyéthylène glycol, ou des mélanges de ceux-ci.

6. Formulation pharmaceutique pour son utilisation selon l'une quelconque des revendications 1 à 5, dans laquelle la phase liquide comprend un ou plusieurs lipides.

7. Formulation pharmaceutique pour son utilisation selon l'une quelconque des revendications 1 à 6, dans laquelle la phase liquide comprend de l'eau et/ou un alcool tel que l'éthanol, le glycérol, le propylène glycol et le polyéthylène glycol et/ou un ou plusieurs lipides ou des mélanges de ceux-ci.

8. Formulation pharmaceutique pour son utilisation selon l'une quelconque des revendications 1 à 7, **caractérisée en ce qu'**elle comprend en outre un ou plusieurs agents aromatiques et/ou aromatisants, tels que ceux choisis dans le groupe constitué par les huiles essentielles obtenues par distillations, extractions avec un solvant ou expressions à froid de fleurs, bourgeons, feuilles, tiges, fruits, graines, peaux, écorces ou racines fraiches ou séchées, par exemple l'huile de menthe poivrée, menthe verte, eucalyptus, thé de bois, niaouli, clou de girofle, cardamome, cannelle, amande amère, coriandre, cumin, gingembre, genièvre, orange, orange amère, citron, pamplemousse, mandarine, bergamote, thym, fenouil et romarin ;
les arômes et les agents générateurs d'arôme naturels comprenant des solutions diluées d'huiles essentielles ou des concentrés de composants d'arôme d'origine naturelle provenant par exemple de fruits, baies, noix, épices, menthes, tabac, cacao, café, thé, vanille, réglisse, caramel, caramel au beurre, miel, vins, liqueurs et bières ;
les arômes et les agents générateurs d'arôme synthétiques se composant de mélanges de produits chimiques comprenant des hydrocarbures, alcools, aldéhydes, esters, cétones, éthers et oxydes mélangés pour être identiques à l'arôme naturel, par exemple de fruits, baies, noix, épices, menthes, tabac, cacao, café, thé, vanille, réglisse, caramel, caramel au beurre, miel, vins, liqueurs ou bières ;
et des mélanges de ceux-ci.

9. Formulation pharmaceutique pour son utilisation selon l'une quelconque des revendications 1 à 8, **caractérisée en ce qu'**elle comprend en outre un ou plusieurs additifs stabilisants, tels que ceux choisis dans le groupe constitué par les antioxydants comprenant la vitamine E, c'est-à-dire les tocophérols, la vitamine C, c'est-à-dire l'acide ascorbique et ses sels, le pyrosulfite de sodium, le butylhydroxytoluène, l'hydroxyanisole butylé ; et conservateurs comprenant les parabènes, le chlorure de benzalconium, le chlorobutanol, l'alcool benzylique, l'alcool β-phényl-éthylique, le chlorure de cétylpyridinium ; et agents chélatants tels que l'EDTA ; et gallates, tels que le gallate de propyle.

10. Formulation pharmaceutique pour son utilisation selon l'une quelconque des revendications 1 à 9, **caractérisée en ce qu'**elle comprend en outre un ou plusieurs additifs choisis dans le groupe constitué par :
les agents épaississants, les exhausteurs, les vitamines, les minéraux, les agents antiodeur, les agents propulseurs, les édulcorants incluant un ou plusieurs agents édulcorants synthétiques et/ou sucres naturels, tels que ceux choisis dans le groupe constitué par les édulcorants artificiels, les polyols, les disaccharides et leurs mélanges.

11. Formulation pharmaceutique pour son utilisation selon l'une quelconque des revendications 1 à 10, **caractérisée en ce qu'**un ou plusieurs des composés de la formulation pharmaceutique sont solubilisés dans un ou plusieurs agents tensioactifs et/ou émulsifiants tels que les tensioactifs non ioniques, cationiques, anioniques ou zwittérioniques, incluant les copolymères séquencés amphiphiles, ou leurs mélanges.

12. Formulation pharmaceutique pour son utilisation selon l'une quelconque des revendications 1 à 11, **caractérisée en ce qu'**elle comprend une base de nicotine libre, de l'hydrogénocarbonate de sodium agissant en tant qu'agent tampon, de l'eau agissant en tant que solvant, de l'éthanol agissant en tant que co-solvant, un poloxamère agissant en tant qu'agent tensioactif, de l'EDTA agissant en tant qu'agent chélatant, de l'acésulfame K agissant en tant qu'édulcorant, facultativement un ou plusieurs conservateurs, et facultativement un ou plusieurs arômes ou agents générateurs d'arôme.

13. Formulation pharmaceutique pour son utilisation selon l'une quelconque des revendications 1 à 12, dans laquelle le traitement offre une réduction rapide et/ou totale du désir de fumer ou d'utiliser du tabac et/ou offre une sensation de contentement procurée par le fait de fumer sans fumer.

14. Formulation pharmaceutique liquide pour son utilisation selon l'une quelconque des revendications 1 à 13, dans laquelle la formulation comprend de la nicotine en une concentration telle que la quantité de nicotine administrée à chaque administration est de 0,05 à 10 mg.

15. Formulation pharmaceutique liquide pour son utilisation selon la revendication 14, dans laquelle la quantité de nicotine administrée à chaque administration est de 0,25 à 6 mg.

16. Formulation pharmaceutique liquide pour son utilisation selon la revendication 14, dans laquelle la quantité de nicotine administrée à chaque administration est de 0,5 à 4 mg.
